# EUROPÄISCHE PATENTSCHRIFT

(11) **EP 1 244 442 B1**
(45) Veröffentlichungstag und Bekanntmachung des Hinweises auf die Patenterteilung: **15.09.2004**
(21) Anmeldenummer: 00993378.9
(22) Anmeldetag: 30.11.2000
(51) Int. Cl.: A61K 31/275, G01N 33/50, A61P 37/00

(54) **VERFAHREN ZUR AUFFINDUNG VON NUKLEOTIDSYNTHESEINHIBITOREN MIT WENIGER NEBENWIRKUNGEN**
METHOD FOR DETECTING NUCLEOTIDE SYNTHESIS INHIBITORS HAVING FEWER SIDE EFFECTS
PROCEDE DE DETECTION D'INHIBITEURS DE SYNTHESE NUCLEOTIDIQUE AYANT MOINS D'EFFETS SECONDAIRES

(30) Priorität: 15.12.1999 DE 19960443
(43) Veröffentlichungstag der Anmeldung: 02.10.2002
(73) Patentinhaber: Aventis Pharma Deutschland GmbH, 65929 Frankfurt am Main (DE)
(72) Erfinder: LINDNER, Jürgen, 35041 Marburg (DE); HAASE, Burkhard, 65719 Hofheim (DE)
(86) Internationale Anmeldenummer: PCT/EP2000/012043
(87) Internationale Veröffentlichungsnummer: WO 2001/043738

(56) Entgegenhaltungen:
- DE-A- 4 127 737
- SILVA ET AL.: "Pharmacokinetik/Pharmacodynamic (PK/PD): Strategy to optimize adminstration, efficacy and safety of the malonitrilamides (MNA) A77 1726, HMR1279 and HMR1715" J. HEART LUNG TRANSPLANTATION, Bd. 17, 1998, Seite 80 XP000995166
- WANG A X ET AL: "Synthesis and immunosuppressant activity of pyrazole carboxamides" BIOORGANIC & MEDICINAL CHEMISTRY LETTERS,GB,OXFORD, Bd. 8, Nr. 19, 6. Oktober 1998 (1998-10-06), Seiten 2787-2792, XP004139621 ISSN: 0960-894X
- SILVA H T ET AL: "Single- and multiple-dose pharmacokinetics and pharmacodynamics of leflunomide's active metabolite A77 1726 in normal Lewis rats." TRANSPLANTATION PROCEEDINGS, (1996 DEC) 28 (6) 3092-4. , XP000994876
- SILVA H.T. ET AL: 'LEFLUNOMIDE AND MALONONITRILAMIDES' THE AMERICAN JOURNAL OF THE MEDICAL SCIENCES Bd. 313, Nr. 5, 1997, Seiten 289 - 301

## Beschreibung

Die Erfindung betrifft ein Verfahren zum Auffinden von Nukleotidsyntheseinhibitoren, die geringere Nebenwirkungen aufweisen.

Ein grundlegendes Problem neuer Medikamente besteht in der Relation zwischen Verträglichkeit und Wirksamkeit. Je größer der Abstand zwischen einer wirksamen Dosis zu einer Dosis ist, bei der Nebenwirkungen auftreten (= therapeutisches Fenster), um so unbedenklicher ist die Anwendung einer derartigen Substanz und um so besser ist die Substanz für den Patienten verträglich.
Cytostatika, zu denen die Purin- und Pyrimidinsyntheseinhibitoren zählen, haben nach bisheriger Erfahrung ein relativ enges therapeutisches Fenster, da sie aufgrund ihrer Hauptwirkung (Unterdrückung der Proliferation von Zellen) auch Nebenwirkungen induzieren. Diese Eigenschaft stört beim Einsatz in onkologischen Indikationen weniger als bei der Therapie von immunologisch bedingten Erkrankungen. Daher wird der Einsatz derartiger Wirkstoffen bei immunologischen Erkrankungen erheblich eingeschränkt.
Verbindungen, welche die Purin- oder Pyrimidinsynthese inhibieren werden als Nukleotidsyntheseinhibitoren bezeichnet (Burkhardt und Kalden; Rheumat. Int. (1997); 17: 85-90), dies sind beispielsweise Verbindungen wie N-(4-Trifluormethylphenyl)-5-methylisoxazol-4-carboxamid, N-(Trifluormethylphenyl)-2-cyan-3-hydroxycroton-säureamid, 2-Cyano-3-cyclopropyl-3-hydroxy-acrylsäure(4-cyanophenyl)-amid oder N-(Trifluormethylphenyl)-2-cyan-3-hydroxy-hept-2-en-6-in-carbonsäureamid, Brequinar (6-Fluor-2-(2'-fluoro[1,1'biphenyl]-4-yl)-3-methyl-4-quinolincarbonsäure), Mycophenolatmofetil (E)-6-(1,3-dihydro-4-hydroxy-6-methoxy-7-methyl-3-oxoisobenzofuran-5-yl)-4-methyl-4-hexenoat), Methotrexat (CAS-Nr. 59-05-02) oder Mizoribine (CAS-Nr. 50924-49-7). In der Publikation von Tedesco et al. (American J Medical Sciences, 1997, 313:, Seiten 289-301) wird bereits vorgeschlagen aktive Analoge von A771726 mit kürzerer Halbwertzeit aus pharmakokinetischen Gründen aufzufinden.

Nukleotidsyntheseinhibitoren eignen sich zur Behandlung von immunologisch bedingten Erkrankungen wie rheumatoider Arthritis oder Pemphigus vulgaris.
Nachteile der bekannten Nukleotidsyntheseinhibitoren sind Nebenwirkungen auf den Gastrointestinaltrakt, Knochenmark, Haare und andere. Geringe Nebenwirkungen bei diesen Verbindungen werden häufig erst sehr spät in der klinischen Entwicklung eines neuen Medikaments entdeckt, welches manchmal zum Abbruch der Entwicklung führt.

Die Erfindung bezweckt von der Beobachtung Gebrauch zu machen, dass man Nebenwirkungen von Nukleotidsyntheseinhibitoren bei der Behandlung von immunologischen Erkrankungen drastisch reduzieren kann, indem die Nukleotidsytheseinhibitoren im Organismus nur kurzzeitig ihre intrazellulär gelegenen Targets erreichen und dort innerhalb kurzer Zeit wieder auf Konzentrationen absinken, die nicht mehr die Nukleotidbiosynthese inhibieren. Durch diese Maßnahme ist es bei der Behandlung immunologischer Erkrankungen möglich, die Nebenwirkungen von Nukleotidsyntheseinhibitoren drastisch zu reduzieren ohne Einbußen bei der erwünschten Wirkung auf das Immunsystem hinnehmen zu müssen.

Mit dieser Kenntnis ist es möglich innerhalb kurzer Zeit Nukeotidsyntheseinhibitorer zu entwickeln, welche bei der Behandlung von immunologisch bedingten Erkrankungen eine sehr gute Wirksamkeit bei einer sehr niedrigen Nebenwirkungsrate zeigen.

Aufgabe der Erfindung ist es bereits in einer frühen Entwicklungsphase von Substanzen in der Arzneimittelforschung an Hand einer technisch leicht meßbaren Eigenschaft eine Aussage über das zu erwartende therapeutische Fenster dieser Substanzen bei immunologischen Indikationen treffen zu können.
Die Aufgabe wird, dadurch gelöst, dass die Halbwertzeit des Nukleotidsyntheseinhibitors im Blut eines für die pharmakokinetischen Verhältnisse beim Menschen relevanten Säugetieres oder beim Menschen in der Phase I der klinischen Entwicklung bestimmt wird und mit der eines bekannten Nukleotidsyntheseinhibitors verglichen wird.

Die Erfindung betrifft daher ein Verfahren zum Auffinden von Nukleotidsyntheseinhibitoren, die geringere Nebenwirkungen aufweisen, das dadurch gekennzeichnet ist, dass man
a) den Nukleotidsyntheseinhibitor einem für die human Situation relevanten Säugetier mit Ausnahme des Menschen in einer wirksamen Dosis appliziert,
b) die Konzentration des Nukleotidsyntheseinhibitors im Blut des Säugetiers feststellt und
c) bestimmt, ob der Nukleotidsyntheseinhibitor eine Halbwertzeit aufweist, die kürzer als die von N-(4-Trifluormethylphenyl)-2-cyan-3-hydroxycrotonsäureamid ist.

Nukleotidsyntheseinhibitoren sind Verbindungen, welche die Purin- oder Pyrimidinsynthese in vivo inhibieren. Unter dem Begriff "Halbwertzeit" wird die Zeit verstanden, in der sich die Konzentration des Nukleotidsyntheseinhibitors im Blutplasma halbiert.

Geeignete Säugetiere zur Durchführung von pharmakokinetischen Vergleichsuntersuchungen sind beispielsweise Mäuse, Ratten, Kaninchen, Hunde, Affen oder Schweine.
Die Halbwertzeiten sind je nach eingesetztem Tier und Nukleotidsyntheseinhibitor sehr unterschiedlich. Die Halbwertzeit von N-(4-Trifluormethylphenyl)-2-cyan-3-hydroxycrotonsäureamid beträgt beispielsweise in der Ratte etwa 4 bis 8 Stunden und beim Menschen bis zu 350 Stunden. Bevorzugt sind Nukteotidsyntheseinhibitoren, die im Blutplasma des Menschen eine Halbwertzeit von weniger als 150 Stunden, bevorzugt weniger als 40 Stunden, aufweisen.

Der Vorteil vom vorliegenden Verfahren ist, dass zu einem sehr frühen Stadium in der Arzneistoffentwicklung über die Messung der Halbwertzeit im Blutplasma schnell eine Aussage über das zu erwartende therapeutische Fenster eines Nukleotidsynthesinhibitors gemacht werden kann. Während sonst erst durch umfangreiche klinische Untersuchungen mit einer Therapiedauer von Wochen bis Jahren der klinisch nutzbare Dosisbereich eines Medikaments erkannt wird. Daher ist eine erhebliche Beschleunigung der Arzneistoffentwicklung möglich.

In nachfolgenden Beispielen wird belegt, dass für die erwünschte Wirkung von Nukleotidsyntheseinhibitoren auf das Immunsystem, Substanzen mit kurzer Halbwertszeit gegenüber Substanzen mit langer Halbwertszeit zu bevorzugen sind:

### Beispiel 1

Dosis-Wirkungsbeziehung von N-(4-Trifluormethylphenyl)-2-cyan-3-hydroxycrotonsäureamid (im folgenden Verbindung 1)

Die Dosis-Wirkungsbeziehung von Verbindung 1 wurde im Modell der Adjuvant induzierten Arthritis (Methode von Pearson C.M. und Wood F.D.; Arth. Rheum. 2:, 44 (1959) und Taurog J.D., Argentieri D.C. et al., Meth. Enzymol. 162: 339 (1988)) erstellt. Bestimmt wurde die Schwellung der Pfoten, die Ausbildung von generalisierten Arthritissymptomen (Scoreindex), die Zunahme des Körpergewichts während des Versuchs und das Überleben der Tiere.
Die Verbindung 1 wurde in einer 1 %-igen Carboxymethylcelluloselösung (CMC) per os von Beginn des Experiments an bis zum Ende am Tag 21 verabreicht. Die Kontrollgruppen erhielten entsprechende Mengen an 1 % CMC ohne Wirkstoff. 6 männliche Lewis Ratten mit einem Körpergewicht von etwa 210g wurden in jeder Versuchsgruppe eingesetzt.

Tabelle 1 zeigt die Ergebnisse nach 21 Tagen, negative Zahlen geben die Verschlechterung des jeweiligen Meßparameters an.

**Tabelle 1:**

| Behandlungsgruppe: | Reduktion Pfotenschwellung | Reduktion Arthritis-Score | Zunahme Körpergewicht | Überlebende Tiere |
|---|---|---|---|---|
| Gesunde Kontrolltiere | - | - | 41 % | 100% |
| Arthritis Kontrolle | 0% | 0% | 12% | 100% |
| Versuchsgruppen Verbindung 1 (mg/kg/Tag) | | | | |
| 1 | -9% | -11% | 10% | 100% |
| 2,5 | 78% | 70% | 20% | 100% |
| 5 | 96% | 83% | 37% | 100% |
| 7,5 | 105% | 87% | 25% | 100% |
| 10 | 96% | 84% | 11% | 100% |
| 15 | 106% | 91% | 8% | 100% |
| 25 | 114% | 96% | -2% | 100% |

Im Modell der Adjuvant induzierten Arthritis in der Ratte werden Autoimmunreaktionen beobachtet, welche den Autoimmunerkrankungen aus dem rheumatischen Formenkreis beim Menschen entsprechen.
In dem Experiment zeigte sich, dass Verbindung 1 bereits ab einer einmaligen täglichen Dosis von 2,5 mg/kg eine sehr gute therapeutische Wirkung in der Ratte zeigt. Unterhalb einer Dosierung von 2,5 mg/kg nimmt die Wirkung drastisch ab. Bei 1 mg/kg/Tag Verbindung 1 kann keine Wirkung mehr festgestellt werden.

### Beispiel 2

### Blutplasmaspiegel von Verbindung 1 in der Ratte

Zur Detektion der Verbindung 1 wurde eine selektive HPLC -MS/MS Methode verwendet. Die quantitative Bestimmung der Verbindung 1 aus dem Blutplasma wurde wie folgt durchgeführt:
Dem entnommenen Blutplasma wurden zugesetzt:
   Lösung eines internen Standards, dann 0,1 m NaOH-Lösung und 1 ml Diethylether. Die Mischung wurde kräftig geschüttelt und die organische Phase verworfen. Zur wäßrigen
   Phase wurde 0,5 N HCl - Lösung zugesetzt und der Analyt sowie der Interne Standard mit 1 ml Diethylether extrahiert. Die organische Phase wurde eingedampft und in einem 1:1 Gemisch aus Acetonitril und Wasser aufgenommen. 10 µl dieser Lösung wurden auf eine HPLC-Anlage injiziert.
   Diese HPLC- Anlage enthielt eine Lichrospher C-18HD Säule. Mit einer Flußrate von 1 ml/min wurden der Analyt und der interne Standard mit der Laufmittelkombination Acetonitril + (Ammoniumacetatlösung [10 mmolar]), 67 + 33, eluiert und anschließend selektiv massenspektroskopisch detektiert (LC-MS / MS - Analytik). Während der Analytik der Blutplasmaproben wurden Kalibrierproben mitgeführt.
   Um eine gleiche fremdstoffmetabolische Situation vorzufinden, wie bei pharmakodynamischen Experimenten, wurde Verbindung 1 nur Tieren mit Adjuvant Arthritis gegeben. Die Arthritis wurde entsprechend Beispiel 1 induziert. Das Tiergewicht betrug im Mittel 210 g, jede Gruppe bestand aus 6 männlichen Lewis Ratten. Blut wurde durch Kupieren der Schwanzspitze gewonnen.
   Untersucht wurde die Beeinflussung der Blutplasmaspiegel von Verbindung 1 durch Colestyramin. Verbindung 1 wurde in der niedrigsten Dosierung von 2,5 mg/kg/Tag eingesetzt, die noch pharmakodynamische Effekte auf die Adjuvant Arthritis zeigt. Bei der Kombinationstherapie mit Colestyramin wurden 1000 mg/kg Colestyramin 4 Stunden nach Gabe der Verbindung 1 appliziert. Kontrolltiere bekamen 1 % CMC zu den entsprechenden Zeitpunkten ohne Wirkstoff. Untersucht wurde die Beeinflussung der Pharmakokinetik der Verbindung 1 durch Colestyramin sowohl nach einmaliger Gabe als auch nach chronischer Gabe der Verbindung 1 mit oder ohne Colestyramin. Blutproben wurden 2, 4, 6, 8, 24 und 48 Stunden nach der letzten Gabe der Verbindung 1 gewonnen.

Tabelle 2 a) zeigt pharmakokinetische Parameter der Verbindung 1 mit und ohne Colestyramin nach einmaliger Gabe; Tabelle 2 b) zeigt pharmakokinetische Parameter der Verbindung 1 mit und ohne Colestyramin nach chronischer Gabe:

**Tabelle 2 a):**

| | 2,5 mg/kg Verbindung 1 | 2,5 mg/kg Verbindung 1 und 1000 mg/kg Colestyramin |
|---|---|---|
| Halbwertszeit [Stunden] | 3,85 | 3,32 |
| Area under the Curve 0-48 Stunden [µg/ml*Std] | 141,69 | 99,90 |
| Maximaler Blutplasmaspiegel [µg/ml] | 10,15 | 9,62 |

Unter dem Begriff "Area under the Curve" (AUC) versteht man die Fläche unter den zeitabhängig aufgezeichneten Konzentrationsspiegel einer Arzneisubstanz. Die AUC reflektiert das Ausmaß mit dem ein Organismus einer Substanz ausgesetzt ist. Unter dem Begriff "chronischen Gabe" versteht man eine Applikation der Verbindung 1 über einen Zeitraum, der zumindest zu konstanten Blutspiegeln der Verbindung 1 führt. Diese konstanten Blutspiegel ("steady state") werden nach regelmäßiger Einnahme nach etwa 4 bis 5 Halbwertszeiten erreicht.

**Tabelle 2 b):**

| | 2,5 mg/kg Verbindung 1 | 2,5 mg/kg Verbindung 1 und 1000 mg/kg Colestyramin |
|---|---|---|
| Halbwertszeit [Stunden] | 3,45 | 3,47 |
| Area under the Curve 0-48 Stunden [µg/ml*Std] | 63,58 | 37,81 |
| Maximaler Blutplasmaspiegel [µg/ml] | 5,73 | 4,66 |

Es zeigte sich, dass nach Gabe von Verbindung 1 zunächst bis zum Zeitpunkt 4 Stunden identische Peakblutspiegel erzielt werden, unabhängig davon, ob nach 4 Stunden Colestyramin gegeben wurde oder nicht. Durch die Gabe von Colestyramin wurden jedoch die Blutspiegel der Verbindung 1 zu späteren Zeitpunkten drastisch reduziert.
Aus diesem Experiment kann geschlossen werden, dass man durch Gabe von Colestyramin die Blutspiegel der Verbindung 1, nach einer vorher ungestörten Resorptionsphase deutlich absenken kann. Die Area under the Curve (AUC) wird um 30% bei Einfachgabe und um 40% bei Mehrfachgabe reduziert.

### Beispiel 3

In Beispiel 3 wurde untersucht, ob die Wirkung der minimal wirksamen Dosierung der Verbindung 1 durch Gabe von Colestyramin im zeitlichen Abstand von 4 Stunden reduziert wird. Untersucht wurde dies im Modell der Adjuvant induzierten Arthritis. Das Modell wurde entsprechend Beispiel 1 durchgeführt. Verbindung 1 wurde täglich von Beginn bis Ende der Untersuchungen in einer Dosierung von 2,5 mg/kg/Tag in 1% Carboxymethylcellulose oral appliziert. Colestyramin wurde 4 Stunden nach der Gabe von Verbindung 1 ebenfalls oral in 1 % Carboxymethylcellulose verabreicht. Die entsprechenden Kontrollgruppen erhielten zu den entsprechenden Zeiten gleiche Volumina an einer 1 % Carboxymethylcelluloselösung. Tabelle 3 zeigt die Ergebnisse:

**Tabelle 3:**

| Behandlungsgruppe | Reduktion Pfotenschwellung | Reduktion Arthritis-Score | Zunahme Körpergewicht | Überlebende Tiere |
|---|---|---|---|---|
| Gesunde Kontrolle | - | - | 30% | 100% |
| Arthritis Kontrolle | 0% | 0% | 17% | 100% |
| 1000 mg/kg/Tag Colestyramin | -14% | -3% | 22% | 100% |
| 2,5 mg/kg/Tag Verbindung 1 | 78% | 70% | 20% | 100% |
| 2,5 mg/kg/Tag Verbindung 1 und 1000 mg/kg/Tag Colestyramin | 86% | 81% | 20% | 100% |

In Beispiel 3 wurde untersucht, ob für die erwünschten Effekte von der Verbindung 1 auf das Immunsystem über längere Zeit bestehende relativ konstante Blutspiegel verantwortlich sind, oder ob kurzzeitig auftretende Blutspiegel (Peakblutspiegel) von Verbindung 1 diesen Effekt bedingen.

Hierzu wurde Verbindung 1 in der niedrigsten wirksamen Dosierung von 2,5 mg/kg/Tag (siehe Beispiel 1) einmal täglich oral appliziert. In den entsprechenden Vergleichsgruppen wurden 4 Stunden nach Gabe der Verbindung 1 Colestyramin appliziert, um die Blutspiegel der Verbindung 1 nach erfolgter Resorptionsphase beschleunigt abzusenken. Hiermit wurde die Wirkung von unterschiedlich lange Zeit bestehenden Blutspiegeln der Verbindung 1 auf die immunologisch bedingten pathologischen Prozesse in der Adjuvant induzierter Arthritis der Ratte untersucht. Es zeigte sich kein Unterschied in der erwünschten Wirkung der Verbindung 1 auf die Adjuvant induzierte Arthritis, unabhängig davon, ob Colestyramin 4 Stunden nach der Gabe von Verbindung 1 gegeben wurde oder nicht.

Da bei der geringsten wirksamen Dosis der Verbindung 1 die Reduktion der Blutspiegel von 4 Stunden nach Applikation zu keiner Reduktion der Wirkung der Verbindung 1 führt, kann gefolgert werden, dass für die Wirkung lediglich die Peakblutspiegel verantwortlich sind und nicht persistierende Blutspiegel der Verbindung 1.

Mit den Beispielen 2 und 3 wurden die Beobachtungen aus der Klinik an Menschen bestätigt (siehe Beispiel 4), dass für die Wirkung von N-(4-Trifluormethylphenyl)-5-methylisoxazol-4-carboxamid (Leflunomide) nur kurzfristige Peakblutspiegel benötigt werden und persistierende Blutspiegel nichts zur Wirksamkeit von Leflunomide beitragen.

### Beispiel 4

Bei einem Patienten, der an der immunologisch bedingten Erkrankung, Pemphigus vulgaris, litt, waren zwar die Symptome dieser Autoimmunerkrankung unter der Therapie mit N-(4-Trifluormethylphenyl)-5-methylisoxazol-4-carboxamid (Leflunomide) vollständig verschwunden, jedoch litt der Patient unter permanenter Diarrhöe. Diese Diarrhöen beeinflußten die Lebensqualität negativ. Aufgrund dieser Nebenwirkungsproblematik wurde nach einer mehr als zweijährigen Therapie mit einer konstanten Dosis von 20mg Leflunomide pro Tag versucht, die Therapie zu beenden. Hierbei wurde beobachtet, dass die Autoimmunerkrankung Pemphigus vulgaris 4 Tage nach dem Absetzen von Leflunomide wieder massiv auftrat. Innerhalb dieses Zeitraumes verschwanden jedoch nicht die Nebenwirkungen der Leflunomidetherapie.
Diese Beobachtung war überraschend, da der aktive Metabolit von Leflunomide, die Verbindung 1, beim Menschen eine Halbwertszeit von etwa 14 Tagen besitzt. Weiterhin wurden bei diesem Patienten mehrfach die Blutspiegel des aktiven Metaboliten von Leflunomide im relativ hohen Bereich zwischen 70 mg/L und 85 mg/L bestimmt. Untersuchungen an Patienten, die an rheumatoider Arthritis leiden und mit Leflunomide behandelt wurden, zeigten Blutspiegel von über 32 mg/L der Verbindung 1.
Aus diesen Beobachtungen kann geschlossen werden, dass der, bei diesem Patienten vorhandene, relativ langsam abfallende Blutspiegel von Verbindung 1 zwar für die Nebenwirkungen nicht jedoch für die erwünschte Wirkung auf das Immunsystem verantwortlich ist.
Ferner zeigen diese Beobachtungen, dass die erwünschten Wirkungen von Leflunomide auf das Immunsystem durch die kurzzeitigen relativ geringen lokalen Spitzenkonzentration von Leflunomide und/oder der Verbindung 1 nach der Resorption hervorgerufen werden und nicht durch die hohen konstanten Blutspiegel der Verbindung 1.

Aus diesen Beobachtungen ist ersichtlich, dass ein gut verträglicher Nukleotidsyntheseinhibitor nur kurzzeitig seinen Wirkort erreichen sollte, damit er dort seine erwünschten Effekte auf das Immunsystem ausüben kann, ohne dass er zu erheblichen Nebenwirkungen führt. Ein längeres Verbleiben am Wirkort trägt überraschender Weise nicht zu einer Steigerung der Wirksamkeit auf das Immunsystem bei, sondern nur zu einem Anstieg der unerwünschten Nebenwirkungen.
Zu erreichen ist dieser Effekt mit Substanzen, welche wie Leflunomide und/oder Verbindung 1 Inhibitoren der Nukleotidbiosynthese sind, aber im Organismus eine kürzere Verweildauer am Wirkort (Halbwertszeit) aufweisen.

### Beispiel 5

Beispiel 5 zeigt die Verträglichkeit von konstanten Blutspiegeln der Verbindung 1 gegenüber pulsartig auftretenden Blutspiegeln der Verbindung 1. Zur Erzielung konstanter Blutspiegel, wurde den Versuchstieren eine Miniinfusionspumpe subkutan implantiert. Die Mininfusionpumpen enthielten 2 ml einer 15%igen ethanolischen Lösung der Verbindung 1, die kontinuierlich über 28 Tage freigesetzt wurden. Eingesetzt wurden Dosierungen von 2,5, 5, 10 und 25 mg/kg/Tag. Zum Vergleich erhielten Tiere, welche die gleichen täglichen Dosierungen der Verbindung 1 aber oral appliziert erhielten Miniinfusionspumpen implantiert, die jedoch lediglich mit dem Vehikel (15% Ethanol in Wasser) befüllt waren. Tieren, welche die Verbindung 1 über Miniinfusionspumpen erhielten, wurde zusätzlich oral eine 1%ige Carboxymethylcelluloselösung appliziert. Als Verlaufsparameter wurde das Überleben der Tiere genommen. Zusätzlich wurde am Tag 5 und Tag 27 Blut entnommen, um die Blutspiegel der Verbindung 1 zu bestimmen. Tieren mit oraler Applikation der Verbindung 1 wurde 4 Stunden nach Applikation Blut entnommen. Die Gruppengröße betrug 6 Tiere, verwendet wurden männliche Lewis Ratten mit einem Körpergewicht von etwa 210 g. Entsprechend den vorhergehenden Versuchen wurde den Tieren am Tag 5 eine Adjuvant Arthritis induziert.

### Tabelle 4 zeigt die Ergebnisse

**Tabelle 4:**

| Behandlungsgruppe: | Verbindung 1 Blutplasmaspiegel (Tag 5) | Verbindung 1 Blutplasmaspiegel (Tag 27) | Körpergewicht Zunahme | Überlebende Tiere |
|---|---|---|---|---|
| Gesunde Tiere Kontrollgruppe | - | - | 36% | 100% |
| Arthritis Kontrolle | - | - | -4% | 100% |
| 2,5 mg/kg/Tag Verbindung 1 p.o. | Nicht bestimmt | 9,66 µg/ml ± 1,56 | +29% | 100% |
| 5 mg/kg/Tag Verbindung 1 p.o | 14,31 µg/ml ± 2,85 | 18,0 µg/ml ± 3,76 | +25% | 100% |
| 10 mg/kg/Tag Verbindung 1 p.o. | Nicht bestimmt | 34,35 µg/ml ± 4,98 | +27% | 100% |
| 25 mg/kg/Tag Verbindung 1 p.o. | Nicht bestimmt | 80,5 µg/ml ± 6,53 | -2% | 100% |

| Behandlungsgruppe: | Verbindung 1 Blutplasmaspiegel (Tag 5) | Verbindung 1 Blutplasmaspiegel (Tag 27) | Körpergewicht Zunahme | Überlebende Tiere |
|---|---|---|---|---|
| 2,5 mg/kg/Tag Verbindung 1 - Miniinfusion- | 2,05 µg/ml ± 0,48 | 2,34 µg/ml ± 0,58 | +22% | 100% |
| 5 mg/kg/Tag Verbindung 1 - Miniinfusion- | 3,57 µg/ml ± 1,50 | 4,18 µg/ml ± 0,84 | +26% | 100% |
| 10 mg/kg/Tag Verbindung 1 - Miniinfusion- | 4,55 µg/ml ± 2,92 | 6,72 µg/ml ± 1,10 | +29% | 100% |
| 25 mg/kg/Tag | - | - | +13%* | 17% |

| | | | | |
|---|---|---|---|---|
| * in dieser Gruppe überlebte nur 1 von 6 Tieren, Körpergewichtszunahme daher nur bei einem Tier bestimmt; p.o. bedeutet per os. | | | | |

Beispiel 5 zeigt, dass konstante Blutspiegel der Verbindung 1 weniger verträglich sind als kurzzeitig auftretende hohe Blutspiegel.
Zur Erzielung konstanter Blutspiegel von Verbindung 1 wurde den Tieren subkutan eine Miniinfusionspumpe implantiert, welche den Tieren über 28 Tage kontinuierlich Verbindung 1 zuführt. Hierdurch wurden konstante Blutspiegel an Verbindung 1 erzielt und somit lange Halbwertszeiten von Verbindung 1, wie sie beim Menschen beobachtet werden, simuliert.

Als Vergleichsgruppen dienten Tiere, die mit der identischen täglichen Dosis an Verbindung 1 behandelt wurden, die Substanz jedoch nicht per Miniinfusionspumpe sondern oral erhielten. Oral applizierte Verbindung 1 weißt in der Ratte, wie in Beispiel 2 bestimmt, eine kurze Halbwertszeit von etwa 4 bis 8 Stunden auf.

In diesem Experiment wurde die Verträglichkeit der Verbindung 1 mit kurzer Halbwertszeit (Verbindung 1 oral appliziert) mit der Verträglichkeit der Verbindung mit langer Halbwertszeit (Verbindung 1 durch Pumpe mit konstanter unendlicher Halbwertszeit) verglichen. An Dosierungen wurden 2,5 mg/kg/Tag, 5 mg/kg/Tag, 10 mg/kg/Tag und 25 mg/kg/Tag eingesetzt. Als Maß für die Verträglichkeit wurde die Anzahl, der die Behandlung überlebenden Tiere, herangezogen. Verglichen wurde die Verträglichkeit außerdem bezüglich der beobachteten Blutspiegel.

Bei Tieren, die mit der Miniinfusionspumpe 25 mg/kg/Tag Verbindung 1 erhielten, verstarben 93% der Tiere. Im Gegensatz hierzu überlebten alle Tiere, denen die 25 mg/kg/Tag Dosis oral appliziert wurde. Dieses Experiment zeigt, dass konstante Blutspiegel der Verbindung 1 bei gleicher täglicher Substanzbelastung wesentlich schlechter vertragen werden, als kurzfristig auftretende und wieder abnehmende Blutspiegel der Verbindung 1.

Die Blutspiegel der Tiere mit Miniinfusionspumpen waren am Tag 5 nach Beginn des Versuches und am Ende etwa gleich. Die Tiere mit Infusionspumpe zeigte je nach eingestellter Dosierung Blutplasmawerte der Verbindung 1 von 2,34 µg/ml, 4,18 µg/ml und 6,72 µg/ml. Aufgrund des schlechten Zustandes der Tiere in der 25 mg/kg Dosisgruppe konnte den Tieren kein Blut entnommen werde.
Aus den Werten bei niedrigeren Dosierungen kann jedoch extrapoliert werden, dass in der 25 mg/kg/Tag Dosisgruppe, die Verbindung 1 über Miniinfusionspumpe erhielten, die Blutspiegel nicht über 15 µg/ml gelegen haben können.
Die Tiere mit oraler Gabe von Verbindung 1 zeigten wesentlich höhere Blutplasmawerte von 9,66 µg/ml, 18,01 µg/ml, 34,35 µg/ml und 80,50 µg/ml.
Der Vergleich der Blutspiegel zeigt somit, dass Tiere mit oraler Gabe der Verbindung 1 wesentlich höhere Blutspiegel aufwiesen (etwa 4 bis 5 mal höher), als Tiere mit der Miniinfusionspumpe.

Daher zeigt Beispiel 5, dass konstante Blutspiegel der Verbindung 1 wesentlich schlechter verträglich sind als pulsartig auftretende Blutspiegel, selbst wenn diese 4 bis 5 mal höher sind als die konstanten Blutspiegel.
Dieses Ergebnis ist mit der ursprünglichen klinischen Beobachtung in Übereinstimmung, dass die an Menschen beobachteten konstanten Blutspiegel der Verbindung 1 verantwortlich für deren Nebenwirkungen sind.
Beispiel 5 zeigt, dass Substanzen, welche die Wirksamkeit der Verbindung 1 aufweisen aber kürzere Halbwertszeiten zeigen, wesentlich besser verträglich sind als Substanzen mit einer langen Halbwertszeit.

## Patentansprüche

1. Verfahren zum Auffinden von Nukleotidsyntheseinhibitoren, die geringere Nebenwirkungen aufweisen, **dadurch gekennzeichnet, dass** man
a) den Nukleotidsyntheseinhibitor einem Säugetier, wobei der Mensch ausgenommen ist, in einer wirksamen Dosis appliziert,
b) die Konzentration des Nukleotidsyntheseinhibitors im Blut des Säugetiers feststellt und
c) bestimmt, ob der Nukleotidsyntheseinhibitor eine Halbwertzeit aufweist, die kürzer als die von N-(4-Triftuormethylphenyl)-2-cyan-3-hydroxy-crotonsäureamid ist.

2. Verfahren gemäß Anspruch 1, **dadurch gekennzeichnet, dass** ein Säugetier aus der Gruppe Maus, Ratte, Kaninchen, Hund, Affe oder Schwein eingesetzt wird.

## Claims

1. A procedure for finding nucleotide synthesis inhibitors which have slighter side effects, which comprises
a) administering the nucleotide synthesis inhibitor in an efficacious dose to a mammal, with the exception of humans,
b) determining the concentration of the nucleotide synthesis inhibitor in the blood of the mammal and
c) determining whether the nucleotide synthesis inhibitor has a half-life which is shorter than that of N-(4-trifluoromethylphenyl)-2-cyano-3-hydroxycrotonamide.

2. The procedure as claimed in claim 1, wherein a mammal from the group consisting of mice, rats, rabbits, dogs, monkeys or pigs is employed.

## Revendications

1. Procédé de détection d'inhibiteurs de la synthèse des nucléotides qui présentent des effets secondaires moindres, **caractérisé en ce que** l'on
a) applique l'inhibiteur de synthèse de nucléotide à un mammifère, exception faite de l'homme, en une dose efficace,
b) établit la concentration de l'inhibiteur de synthèse de nucléotide dans le sang du mammifère et
c) détermine si l'inhibiteur de synthèse de nucléotide présente une demi-vie qui est plus courte que celle de l'amide d'acide N-(4-trifluorométhylphényl)-2-cyano-3-hydroxy-crotonique.

2. Procédé selon la revendication 1, **caractérisé en ce qu'**un mammifère choisi parmi le groupe souris, rat, lapin, chien, singe, cochon est utilisé.
